# FASCICULE DE BREVET EUROPEEN

(11) **EP 0 437 406 B1**
(45) Date de publication et mention de la délivrance du brevet: **31.03.1993**
(21) Numéro de dépôt: 91400054.2
(22) Date de dépôt: 11.01.1991
(51) Int. Cl.: A61K 31/70, A61K 33/42, A61K 33/08

(54) **Composition pharmaceutique sous forme de suspension aqueuse stable à base de sucralfate et d'une substance anti-acide ainsi que son procédé de préparation**
Arzneimittelzusammensetzung in stabiler wässriger Suspensionsform auf der Basis von Sucralfat und einem Antacidum sowie Verfahren zu ihrer Herstellung
Pharmaceutical composition in stable aqueous suspension form containing sucralfate and anti-acidic substance as well as process for preparing it

(30) Priorité: 12.01.1990 FR 9000341
(43) Date de publication de la demande: 17.07.1991
(73) Titulaire: PIERRE FABRE MEDICAMENT, 92100 Boulogne (FR)
(72) Inventeur: Davin, Hervé, F-81660 Pont de l'Arn (FR)
(74) Mandataire: Warcoin, Jacques

(56) Documents cités:
- EP-A- 0 286 978
- WO-A-86/01406
- DE-C- 3 330 944
- FR-A- 2 364 657

## Description

La présente invention concerne une préparation pharmaceutique destinée à l'administration par voie orale, présentée sous forme de suspension stable, en l'absence d'agent viscosant associant le sucralfate à des substances anti-acides, capables de neutraliser l'acidité gastrique, ainsi que leur procédé de préparation.

Le sucralfate est du saccharose sulfate d'aluminium basique. Il est utilisé en médecine humaine dans le traitement des ulcères gastriques et duodénaux. Absorbé à la dose de 0,5g à 2g par jour, le sucralfate agit sur le tractus digestif en recouvrant la muqueuse de l'estomac et du duodenum, d'un gel protecteur.

La formation de ce gel est consécutive à la réaction qui se produit entre le sucralfate et l'acide chlorhydrique du milieu gastrique. Le gel ainsi produit est très adhésif. Il tapisse les parois gastrique et duodénale et en raison du tropisme électromagnétique qu'il présente pour les molécules protéiniques chargées positivement, il forme avec elles un complexe qui isole et protège l'ulcère du contenu gastrique. Par ailleurs, le sucralfate inhibe l'activité protéolytique de la pepsine. Ainsi, il permet et favorise la cicatrisation naturelle de l'ulcère.

Le sucralfate est généralement présenté sous forme sèche telle que comprimés, granulés ou poudres. Il a été également proposé, selon le brevet EP-A-0 136 100 par exemple, des suspensions aqueuses de sucralfate présentées comme aptes à traiter des ulcères aussi bien de la bouche, de l'oesophage et de l'estomac que du duodenum.

Cependant, ces compositions aqueuses renfermant un agent de suspension associé à un adjuvant de suspension, par exemple un dérivé cellulosique associé à du sorbitol, ne présentent pas d'activité améliorée par rapport aux formes sèches précipitées de sucralfate.

Une amélioration des performances a été proposée selon le brevet EP-A-0 286 978, qui consiste à reprécipiter le sucralfate préalablement dissous dans une solution d'acide chlorhydrique; la masse gélatineuse et collante est récupérée par centrifugation, lavée par dispersion et agitation dans l'eau, cette opération étant répétée plusieurs fois afin d'éliminer le chlorure de sodium précédemment formé.

Ce procédé présente plusieurs inconvénients: une manipulation longue, des pertes importantes de sucralfate en raison de la précipitation -qui est partielle- et des lavages successifs. Il se solde par un coût rédhibitoire.

Le procédé qui fait l'objet de la présente invention concerne les formulations de suspensions buvables de sucralfate obtenues directement sans opération de lavage et donc sans perte de principe actif coûteux, associant un agent neutralisant de l'acidité gastrique.

La principale caractéristique de la présente invention réside dans le fait que la substance anti-acide est générée in situ au cours d'une reprécipitation du sucralfate.

Conformément à la présente invention, le procédé de préparation d'une composition pharmaceutique se présentant sous la forme d'une suspension aqueuse stable à base de sucralfate et d'une substance anti-acide, est caractérisé par le fait que l'on effectue les étapes successives suivantes:
* étape de dissolution du sucralfate dans une solution aqueuse acide, puis
* étape de reprécipitation du sucralfate et simultanément de génération in situ de la substance anti-acide, par réaction, sous forte agitation, de la solution de sucralfate obtenue à l'étape précédente, avec une suspension aqueuse d'oxyde, d'hydroxyde et/ou de sel de magnésium, de calcium et/ou d'aluminium, la réaction étant menée jusqu'à obtention d'un pH du milieu réactionnel compris entre 4,3 et 4,9.

La solution aqueuse acide utilisée pour l'étape de dissolution du sucralfate est une solution aqueuse d'acide phosphorique, d'acide citrique, d'acide tartrique, d'acide lactique et/ou d'acide gluconique.

Le sel éventuellement utilisé au cours de l'étape de reprécipitation est un carbonate ou un hydrogénocarbonate.

Ladite substance anti-acide est formée au cours de la préparation de la suspension de sucralfate. Elle intervient dans la phase de solubilisation puis de reprécipitation.

On indiquera, ci-après, à titre d'illustration, un exemple particulier de mise en oeuvre du procédé selon l'invention.

25 g de sucralfate (correspondant à 20g de substance anhydre) sont dissous sous agitation à température ambiante dans 60 ml de solution d'acide phosphorique de concentration comprise entre 10% et 85% en poids. Cette solution est ensuite traitée par une suspension aqueuse d'oxyde de magnésium de concentration comprise entre 0,5% et 20% en poids. L'addition de cette suspension est avantageusement réalisée à température ambiante, sous une très vive agitation afin de disperser et de broyer finement les produits de la réaction. Elle est poursuivie jusqu'à obtention d'un pH compris entre 4,3 et 4,9. Elle peut être menée entièrement ou partiellement par addition de la suspension aqueuse d'oxyde de magnésium. Le cas échéant, le pH est ajusté par addition d'une solution aqueuse d'hydroxyde de sodium de concentration comprise entre 10% et 40% en poids.

La suspension ainsi obtenue se compose de sucralfate reprécipité, sous forme très fine. La taille des particules est de l'ordre de quelques microns.

Dans ce cas particulier, on retrouve, associés au sucralfate, le phosphate de magnésium en suspension, et éventuellement une proportion de phosphate de sodium en solution dans le cas où l'ajustement du pH est terminé à l'aide d'une solution d'hydroxyde de sodium.

Les compositions pharmaceutiques obtenues selon ce procédé présentent diverses caractéristiques nouvelles :
- la présence de sucralfate sous forme de précipité très fin, obtenu directement, sans aucune perte et au terme d'une seule manipulation. La granulométrie particulière du sucralfate renforce ses propriétés physiologiques qui sont liées à la surface élevée du principe actif et à son pouvoir couvrant ;
- la présence de sels doués de propriétés complémentaires de celles du sucralfate, notamment dans le domaine de la thérapeutique anti-ulcéreuse. Ainsi, le phosphate de magnésium est utilisé pour ses propriétés anti-acides, de même que le phosphate de sodium qui agit comme substance tampon ; la présence de ce sel de magnésium doué de propriétés douces, permet de compenser l'action constipante du sucralfate. De plus, le phosphate de magnésium agit comme agent de suspension et améliore les propriétés rhéologiques de la suspension : stabilité, thixotropie et viscosité.

Bien entendu, de façon classique, la suspension ainsi obtenue peut être additionnée de divers autres adjuvants galéniques tel que :
- Edulcorants : tels que sucre, sorbitol glycérine ou saccharose sodique ;
- Arômes : note caramel, lait, vanille ;
- Conservateurs : tels que parahydroxybenzoates, acide sorbique, acide benzoïque.

On observera en outre que les compositions pharmaceutiques obtenues selon le procédé de chimie galénique décrit ci-dessus, présentent outre les avantages techniques et économiques déjà évoqués, l'intérêt de leur activité thérapeutique.

Chez l'animal, les tests d'ulcère de contrainte montrent une augmentation de l'efficacité de ces compositions ; par comparaison avec les formes sèches de sucralfate, le même niveau d'activité anti-ulcéreuse est obtenu avec des doses plus faibles de principe actif (environ 1/2 à 1/3 de la dose).

Chez l'homme, les compositions évaluées dans le cadre de tests cliniques ont montré le même accroissement d'activité. En outre, la composition obtenue avec le couple Acide phosphorique-Oxyde de magnésium a donné le résultat le plus favorable.

Il est interprété de la manière suivante :
- granulométrie fine du sucralfate ;
- effet neutralisant anti-acide de la formule ;
- action de durée prolongée
   qui permettent de réduire la posologie à 2-3 prises de 0,5g de principe actif par jour (au lieu de 3 prises de 1g).
- réduction des phénomènes de constipation, grâce à l'action cholecystokinétique du magnésium ;
- très bonne observance en raison de l'agrément de la commodité d'absorption de la suspension de goût agréable et de caractères organoleptiques appréciés.

## Revendications

1. Procédé de préparation d'une composition pharmaceutique se présentant sous la forme d'une suspension aqueuse stable à base de sucralfate et d'une substance anti-acide, caractérisé en ce que l'on effectue les étapes successives suivantes :
* étape de dissolution du sucralfate dans une solution aqueuse acide, puis
* étape de reprécipitation du sucralfate et simultanément de génération in situ de la substance anti-acide, par réaction, sous forte agitation, de la solution de sucralfate obtenue à l'étape précédente, avec une suspension aqueuse d'oxyde, d'hydroxyde et/ou de sel de magnésium, de calcium et/ou d'aluminium, la réaction étant menée jusqu'à obtention d'un pH du milieu réactionnel compris entre 4,3 et 4,9.

2. Procédé de préparation selon la revendication 1, caractérisé en ce que l'étape de dissolution du sucralfate est réalisée à l'aide d'une solution aqueuse d'acide phosphorique, d'acide citrique, d'acide tartrique, d'acide lactique et/ou d'acide gluconique.

3. Procédé de préparation selon l'une des revendications 1 et 2, caractérisé en ce que le sel utilisé au cours de l'étape de reprécipitation est un carbonate ou un hydrogénocarbonate.

4. Procédé de préparation selon les revendications 1 à 3, caractérisé en ce que la solution aqueuse acide est une solution d'acide phosphorique présentant une concentration de 10 à 85% en poids.

5. Procédé de préparation selon les revendications 1 à 4, caractérisé en ce que l'étape de reprécipitation est effectuée par réaction avec une suspension aqueuse d'oxyde de magnésium présentant une concentration de 0,5 à 20% en poids.

6. Procédé de préparation selon la revendication 5, caractérisé en ce que le pH du milieu réactionnel est ajusté explicitement par addition de la suspension d'oxyde de magnésium.

7. Procédé de préparation selon les revendications 1 à 5, caractérisé en ce que le pH du milieu réactionnel est ajusté par addition d'une solution aqueuse d'hydroxyde de sodium de concentration comprise entre 10 et 40% en poids.

8. Procédé de préparation selon les revendications 1 à 7, caractérisé en ce que l'étape de reprécipitation est effectuée à température ambiante.

9. Procédé de préparation selon les revendications 1 à 8, caractérisé en ce que l'on utilise une solution aqueuse d'acide phosphorique, une suspension aqueuse d'oxyde de magnésium et éventuellement une solution aqueuse d'hydroxyde de sodium.

10. Composition pharmaceutique se présentant sous la forme d'une substance aqueuse stable à base de sucralfate et d'une substance anti-acide, caractérisée en ce que ladite substance anti-acide est générée in situ au cours d'une étape de reprécipitation du sucralfate.

11. Composition pharmaceutique selon la revendication 10, caractérisée en ce que l'agent anti-acide est du phosphate de magnésium éventuellement associé à du sulfate de sodium.

12. Composition pharmaceutique selon l'une des revendications 10 et 11, caractérisée en ce que le sucralfate reprécipité se présente sous la forme de fines particules ayant une taille moyenne de l'ordre de quelques microns.

13. Composition pharmaceutique selon les revendications 10 à 12, caractérisée en ce qu'elle est obtenue par la mise en oeuvre du procédé selon l'une des revendications 1 à 9.

## Patentansprüche

1. Verfahren zur Herstellung einer pharmazeutischen Zusammensetzung, die in Form einer stabilen wäßrigen Suspension vorliegt, auf Basis von Sucralfat und einer antaciden Substanz, dadurch gekennzeichnet, daß man die folgenden aufeinanderfolgenden Stufen durchführt:
- Auflösung des Sucralfats in einer sauren wäßrigen Lösung, dann
- Wiederausfällung des Sucralfats und gleichzeitige in situ-Bildung der antaciden Substanz durch Umsetzung der in der vorhergehenden Stufe erhaltenen Sucralfat-Lösung unter starkem Rühren mit einer wäßrigen Suspension eines Oxids, Hydroxids und/oder Salzes von Magnesium, Calcium und/oder Aluminium, wobei die Reaktion bis zur Erzielung eines pH-Wertes des Reaktionsmediums zwischen 4,3 und 4,9 durchgeführt wird.

2. Herstellungsverfahren nach Anspruch 1, dadurch gekennzeichnet, daß die Stufe der Auflösung des Sucralfats mit Hilfe einer wäßrigen Phosphorsäure-, Zitronensäure-, Weinsäure-, Milchsäure- und/oder Gluconsäurelösung durchgeführt wird.

3. Herstellungsverfahren nach einem der Ansprüche 1 und 2, dadurch gekennzeichnet, daß das in der Stufe der Wiederausfällung verwendete Salz ein Carbonat oder ein Hydrogencarbonat ist.

4. Herstellungsverfahren nach den Ansprüchen 1 bis 3, dadurch gekennzeichnet, daß die saure wäßrige Lösung eine 10 bis 85 gew.-%ige Phosphorsäurelösung ist.

5. Herstellungsverfahren nach den Ansprüchen 1 bis 4, dadurch gekennzeichnet, daß die Stufe der Wiederausfällung durchgeführt wird durch Umsetzung mit einer wäßrigen, 0,5 bis 20 gew.-%igen Magnesiumoxid-Suspension.

6. Herstellungsverfahren nach Anspruch 5, dadurch gekennzeichnet, daß der pH-Wert des Reaktionsmediums durch Zugabe der Magnesiumoxidsuspension ausdrücklich eingestellt wird.

7. Herstellungsverfahren nach den Ansprüchen 1 bis 5, dadurch gekennzeichnet, daß der pH-Wert des Reaktionsmediums durch Zugabe einer wäßrigen, 10 bis 40 gew.-%igen Natriumhydroxidlösung eingestellt wird.

8. Herstellungsverfahren nach den Ansprüchen 1 bis 7, dadurch gekennzeichnet, daß die Stufe der Wiederausfällung bei Umgebungstemperatur durchgeführt wird.

9. Herstellungsverfahren nach den Ansprüchen 1 bis 8, dadurch gekennzeichnet, daß man eine wäßrige Phosphorsäurelösung, eine wäßrige Magnesiumoxidsuspension und gegebenenfalls eine wäßrige Natriumhydroxidlösung verwendet.

10. Pharmazeutische Zusammensetzung, die in Form einer stabilen wäßrigen Substanz vorliegt, auf Basis von Sucralfat und einer antaciden Substanz, dadurch gekennzeichnet, daß die genannte antacide Substanz in der Stufe der Wiederausfällung des Sucralfats in situ gebildet wird.

11. Pharmazeutische Zusammensetzung nach Anspruch 10, dadurch gekennzeichnet, daß es sich bei dem antaciden Agens um das Magnesiumphosphat handelt, das gegebenenfalls mit Natriumsulfat assoziiert ist.

12. Pharmazeutische Zusammensetzung nach einem der Ansprüche 10 und 11, dadurch gekennzeichnet, daß das wiederausgefällte Sucralfat in Form von feinen Teilchen mit einer mittleren Teilchengröße in der Größenordnung von einigen Mikron (µm) vorliegt.

13. Pharmazeutische Zusammensetzung nach den Ansprüchen 10 bis 12, dadurch gekennzeichnet, daß sie erhalten wird durch Anwendung des Verfahrens nach einem der Ansprüche 1 bis 9.

## Claims

1. Process for preparing a pharmaceutical composition in the form of a stable aqueous suspension based on a sucralphate and an antacid substance, characterized in that the following stages are carried out :
* a stage in which the sucralphate is dissolved in an aqueous acid solution, followed by
* a stage in which the sucralphate is reprecipitated and the antacid substance is simultaneously produced in situ by reacting, with vigorous stirring, the sucralphate solution obtained in the previous stage with an aqueous suspension of magnesium, calcium and/or aluminium oxide, hydroxide and/or salt, the reaction being continued until the pH of the reaction medium reaches a value of between 4.3 and 4.9.

2. Preparation process according to Claim 1, characterized in that the stage in which the sucralphate is dissolved is carried out using an aqueous solution of phosphoric, citric, tartaric, lactic and/or gluconic acid.

3. Preparation process according to either of Claims 1 or 2, characterized in that the salt used in the reprecipitation stage is a carbonate or a hydrogen carbonate.

4. Preparation process according to Claims 1 to 3, characterized in that the aqueous acid solution is a phosphoric acid solution having a concentration of 10 to 85% by weight.

5. Preparation process according to Claims 1 to 4, characterized in that the reprecipitation stage is carried out by reaction with an aqueous suspension of magnesium oxide having a concentration of 0.5 to 20% by weight.

6. Preparation process according to Claim 5, characterized in that the pH of the reaction medium is adjusted explicitly by addition of a suspension of magnesium oxide.

7. Preparation process according to Claims 1 to 5, characterized in that the pH of the reaction medium is adjusted by addition of an aqueous solution of sodium hydroxide having a concentration of between 10 and 40% by weight.

8. Preparation process according to Claims 1 to 7, characterized in that the reprecipitation stage is carried out at ambient temperature.

9. Preparation process according to Claims 1 to 8, characterized by the use of an aqueous solution of phosphoric acid, an aqueous suspension of magnesium oxide and possibly an aqueous solution of sodium hydroxide.

10. Pharmaceutical composition in the form of a stable aqueous suspension based on a sucralphate and an antacid substance, characterized in that the said antacid substance is produced in situ during a stage in which the sucralphate is reprecipitated.

11. Pharmaceutical composition according to Claim 10, characterized in that the antacid agent is magnesium phosphate possibly together with sodium sulphate.

12. Pharmaceutical composition according to either of the Claims 10 or 11, characterized in that the reprecipitated sucralphate is in the form of fine particles having an average size of the order of several microns.

13. Pharmaceutical composition according to Claims 10 to 12, characterized in that it is obtained by means of a process given in one of the Claims 1 to 9.
